Europäisches Patentamt

European Patent Office

Office européen des brevets

(19)

(11) Publication number: 0 364 078
A1

## (12) EUROPEAN PATENT APPLICATION

(21) Application number: 89308026.7

(22) Date of filing: 07.08.89

(51) Int. Cl.5: G01N 35/02 , G01N 35/04

(30) Priority: 19.08.88 GB 8819734

(43) Date of publication of application:
18.04.90 Bulletin 90/16

(84) Designated Contracting States:
AT BE CH DE ES FR GB GR IT LI LU NL SE

(71) Applicant: British Sugar plc
PO Box 26 Oundle Road
Peterborough PE2 8QU(GB)

(72) Inventor: Leaton, Peter Robert
59 North Street Stilton
Cambridgeshire PE7 3PP(GB)
Inventor: Rogers, Robin Hugh
12 Hawthorne Close
Market Deeping Lincolnshire(GB)

(74) Representative: Serjeant, Andrew Charles
25 The Crescent King Street
Leicester, LE1 6RX(GB)

(54) Liquid sampling apparatus.

(57) A conveyor (10) transports containers (12) in sequence through fill, mix and sampling stations (14,16,18) respectively. A balance 26 at the fill station (14) determines the weight of matter in the container (12). Means (28) introduces into the container (12) an amount of reagent corresponding to the weight of matter in the container (12). Means are provided for drawing off a sample of the contents of the container (12) at the station (18) for examination, and means for washing and drying the container (12) are provided at subsequent stations (20,22,24). The apparatus is suitable for the determination of sugar and amino nitrogen content in beet delivered to a sugar factory.

FIG.1

Xerox Copy Centre

## Liquid Sampling Apparatus

## Technical Field

The invention relates to liquid samping apparatus suitable for use in an industrial laboratory. The invention is particularly but not exclusively concerned with the preparation of samples of clear solution for the determination of sugar and amino nitrogen content in beet delivered to a sugar factory.

## Background Art

On arrival at a sugar factory, beet is sampled so that payment can be made to the grower on the basis of sugar content. The beet is cleaned, sliced and macerated to give a brown pulp called brei. The brei is treated with basic lead acetate solution, aluminium chloride solution, or aluminium sulphate solution to extract the sugar. The liquid is filtered to produce a clear solution which is passed to a saccharimeter and colourimeter for the determination of sugar and amino nitrogen. The clarification and filtration steps are labour-intensive, and can economically be performed by apparatus according to the invention.

Patent Specification DE 1696229 A relates to apparatus of this kind. Homogeneous brei is fed up to a predetermined quantity by a conveyor into a container on a weighing machine. The container is moved to another weighing machine where water is added up to a ratio between brei and water of 1 to 3. The container is then transferred to a conveyor, and thereby to one of a number of mixers which is free. At the mixer, the container is moved from the conveyor, its contents are mixed, and basic lead acetate is added as a solution. The container is then moved by another conveyor to a filtering station, where the container is removed from the conveyor, and the filtrate syphoned off for polarimetric determination of its sugar content. This apparatus involves the use of two weighing machines, separate handling mechanism for the containers at that stage, two conveyors, and transfer means for removing the containers from the conveyors for mixing and for examination.

Patent Specification US 3481709 relates to automated analytical apparatus suitable for capsules. This includes no balance or weighing machine, and consequently no related means for controlling the amount of reagent added. Here again, two conveyors are used for taking a sample through all the relevant stations.

Other examples of sampling or analysis apparatus are shown in Patent Specification US 3843243 and DE 1907464 A, while the automatic following of samples by means of cards is the subject of FR 2537552.

## The Invention

Apparatus according to the invention comprises a conveyor for transporting containers for liquid through a number of stations in sequence, a balance at a first station for determining the weight of matter in the container, means for introducing into the container an amount of reagent corresponding to the weight of matter, a mixer at a second station for stirring the contents of the container, means for drawing off a sample of the contents of the container at a third station, means for examining the sample, and means for washing and drying the container at subsequent stations.

The conveyor is preferably a twin chain conveyor provided with mountings for securing the containers which may be beakers of stainless steel or other material not corroded by the reagent and capable of withstanding repeated washing and drying. The first, second and third stations are preferably in line, and the washing and drying stations on a return run of the conveyor, preferably below the earlier stations. The conveyor may be advanced intermittently by a control unit taking into account the state of the operations performed at the various stations, the presence of sample being noted by photo-electric cells on an identifying card conveyor.

The balance may be of a kind known in itself and equipped with means for introducing reagent in an amount automatically calculated from the weight of matter in the container, and determined by the increase in weight of the container as the reagent is added.

The mixer may be simply an agitator or rotary paddle wheel which can be lowered into the container, operated while the container is at the second station and then withdrawn.

The means for drawing off a sample from the container preferably includes a filter. The filter may be provided by a piece of filter paper cut from a roll and retained onto the surface of the die by vacuum, before the sample is sucked up through the filter paper and taken away for examination. To this end, the apparatus may include a mounting for a roll of filter paper, means for advancing the filter paper, and cutting a piece of suitable size. Any waste filter paper can be conveyed away towards a drain below the conveyor into which the used filter paper and surplus contents of the container are washed at the washing station.

The sample can be examined in a manner known in itself, for example by passing through a saccharimeter and a colourimeter. A saccharimeter is generally speaking a polarimeter which determines the sugar content of a solution by measuring the extent to which it rotates the plane of polarized light passed through a sample in a standard container. The colourimeter determines the amino nitrogen content of the solution which is an indication of the non-sugars present in the beet, and shows whether nitrogenous fertilizers have been applied to the plants in the correct proportion. The "Blue Number" method may be used employing the reaction of copper nitrate with alpha amino nitrogen compounds to give a complex which can be measured at 600 nm. These meters operate by passing light through a cell or tube of sample, and can be made to give a direct reading using a calculating interface, microcomputer, recorder and/or printer. A single sample may be divided and part examined in each. The sample can then be passed to the drain provided for the apparatus as a whole.

Matter for sampling is generally marked as to its origin with details on a card. The card may be carried around the conveyor in a slot provided for each container. The results of the analysis may be printed on the card, or fed directly to the control unit or a computer where they may be used together with other information to calculate the payment due to a beet grower, for example.

## DRAWINGS

Figure 1 is a side view of apparatus according to the invention with the covers removed;

Figure 2 is a top plan view corresponding to Figure 1;

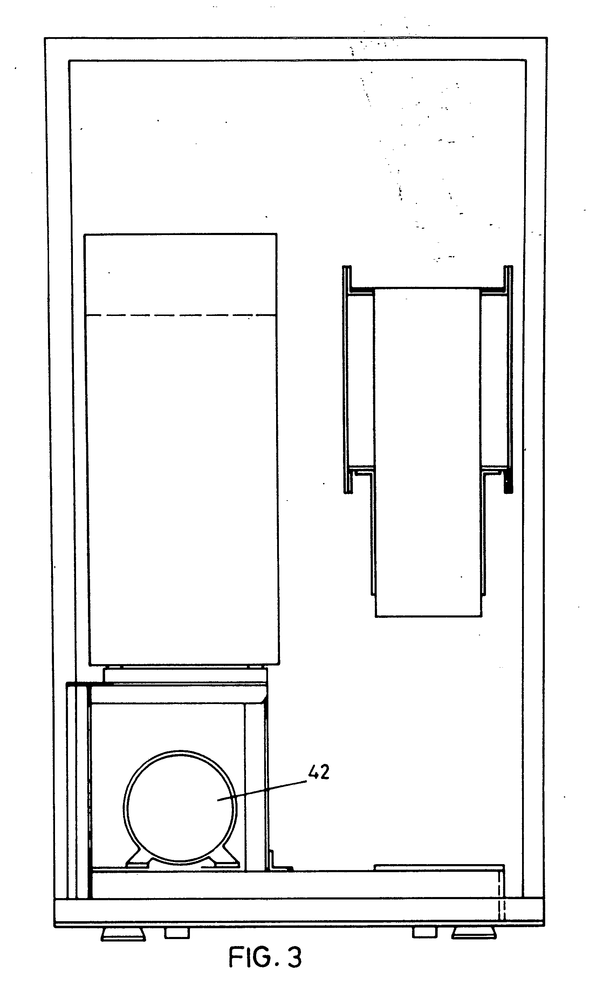

Figure 3 is a corresponding left hand end view of Figure 1;

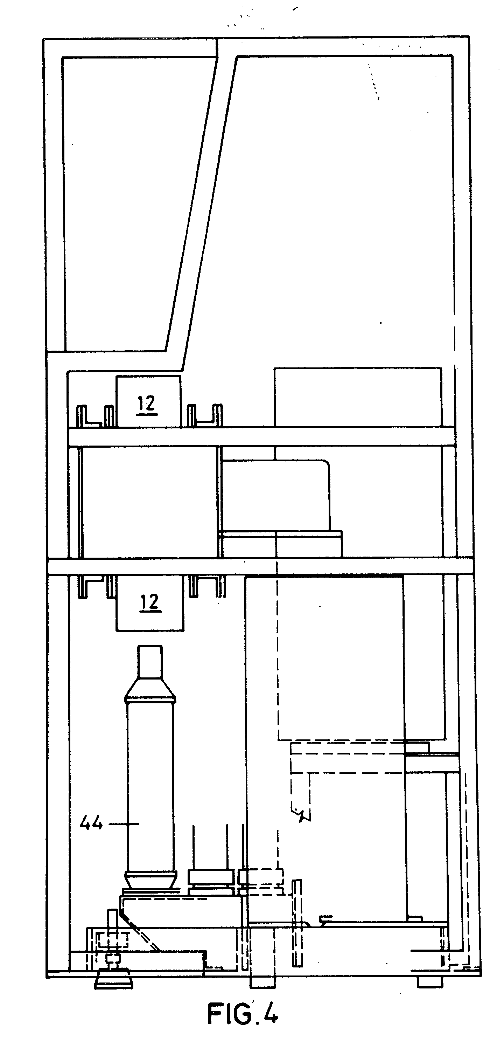

Figure 4 is a corresponding right hand end view of Figure 1;

Figure 5 is an isometric of the apparatus complete with an operator feeding material for sampling;

Figure 6 is a plan corresponding to Figure 5, omitting the operator, but showing also the arrival of matter (brei) for sampling from the right;

Figure 7 is a side view of a conveyor for transporting containers for liquid through the apparatus of Figure 1;

Figure 8 is a plan of the conveyor of Figure 7;

Figure 9 is an end view of the conveyor of Figure 7;

Figure 10 is an exploded view of a balance in the apparatus of Figure 1;

Figure 11 shows means for introducing reagent in Figure 1;

Figure 11a is a horizontal section through reagent introduction nozzles in Figure 11;

Figure 12 shows a mixer in Figure 1;

Figure 13 is a side view of means for drawing off a sample of the contents of a container at a third station in the apparatus of Figure 1.

## Best Mode

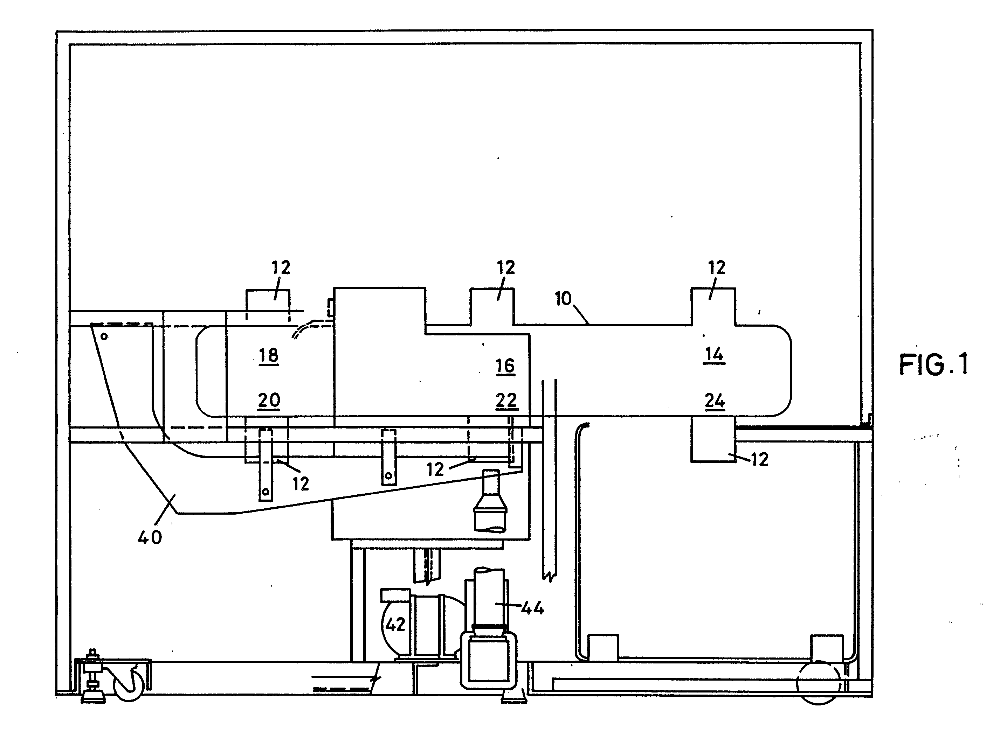

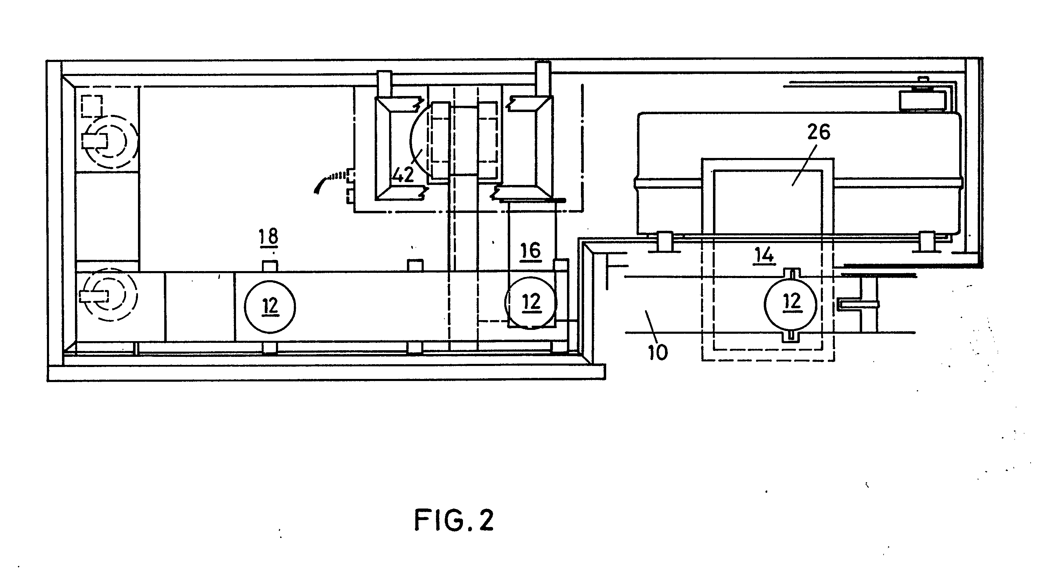

With particular reference to Figures 1 and 2, a conveyor 10 (indicated only schematically in Figure 1) transports containers 12 in sequence through a number of stations. In Figure 1, there are shown a fill station 14, a mix station 16, and a sample station 18 on an upper run of the conveyor 10, and a wash station 20, a hot dry station 22, and a cold dry station 24 on a lower or return run of the conveyor 10.

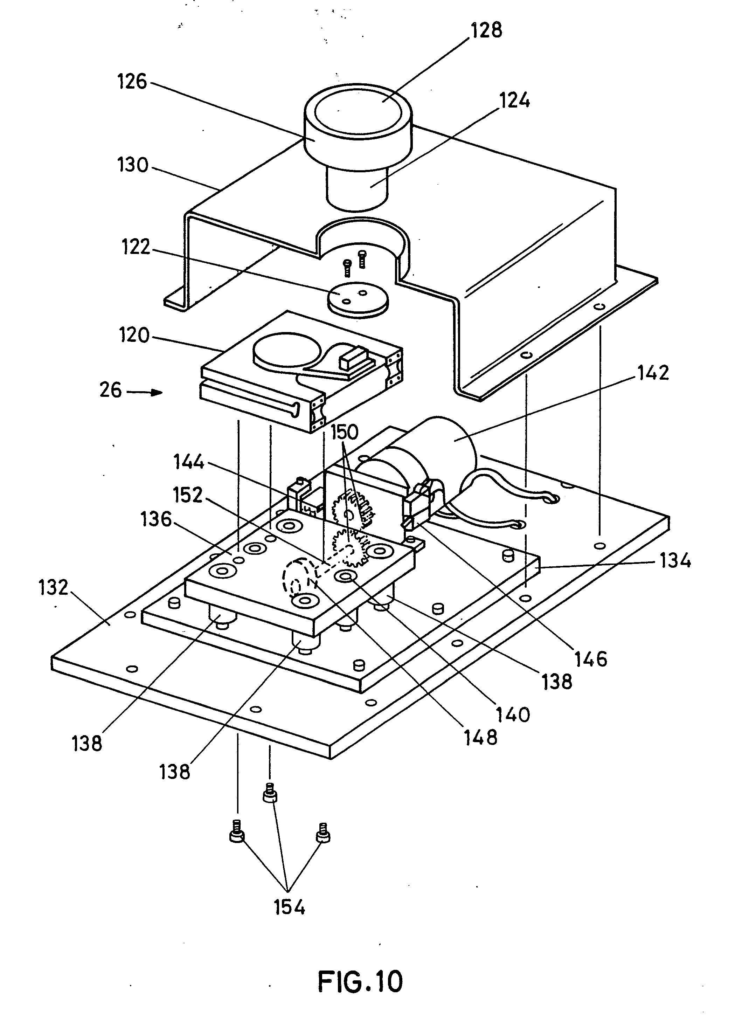

A balance 26 (Figures 2 and 10) is provided at the fill station 14 for determining the weight of matter in the container 12. A control unit (not shown) stops the conveyor 10 with a container 12 at the fill station 14. The operation of the balance is described in relation to Figure 10 below.

At the sample station 18, there is means for drawing off a sample of the contents of the container 12. This sample is passed by means of a pump to a saccharimeter and a colourimeter for examination in a manner known in itself.

At the wash station 20, the container 12 has its aperture facing downward, and its inside is washed by means of a jet of water which carries away any matter remaining in the container 12 into a drain trough 40. At the hot dry station 22, air is blown by an electric motor 42 through a heater 44 into the container 12.

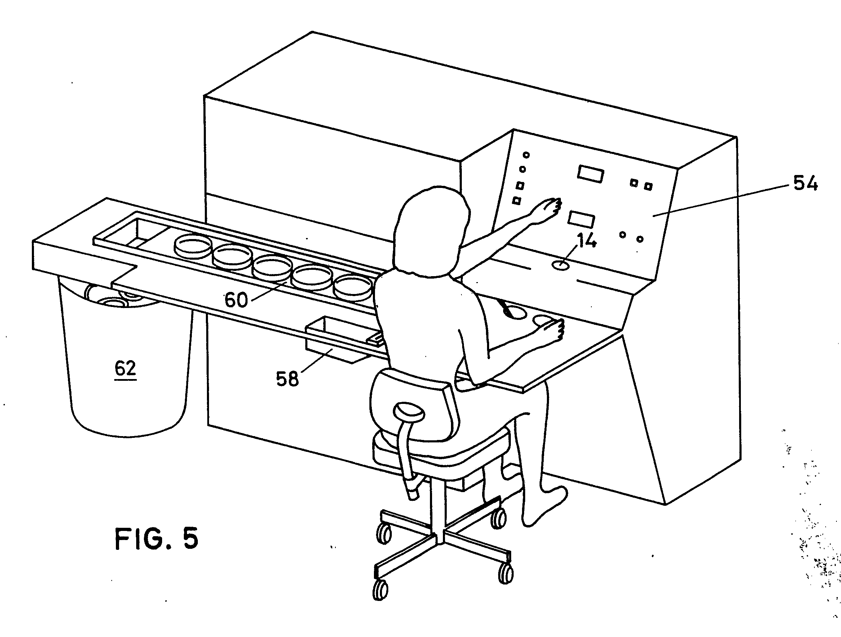

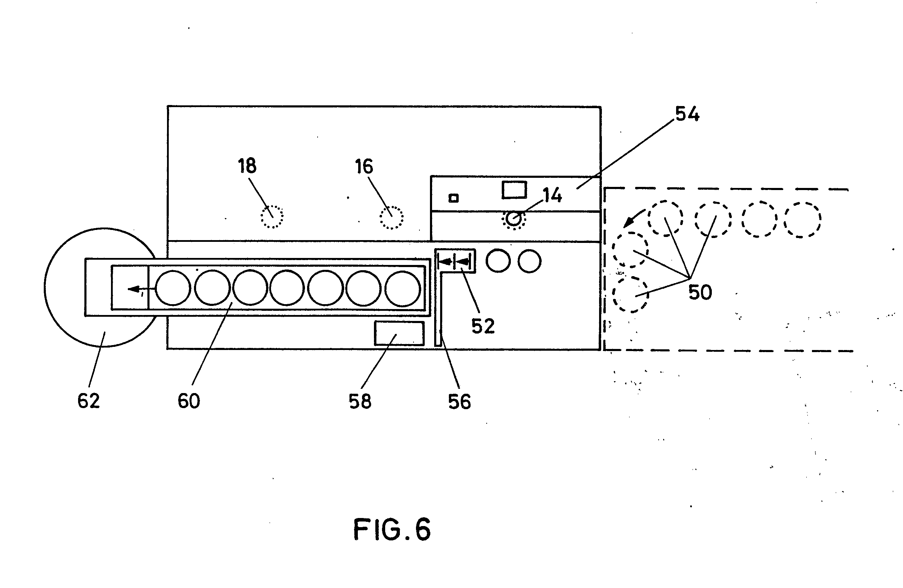

Figure 5 shows the outside of the whole apparatus, while the stations 14,16 and 18 are only indicated in outline because the latter at least are not visible. Figure 6 shows matter (brei) for sampling arriving from the right in pots 50, each accompanied by a card (not shown) with details at to its origin. After placing the sample in the container 12, an operator places the record card in a right hand slot of a card conveyor 52. The card conveyor 52 moves the card in three steps to the left until the appropriate reading from the saccharimeter appears on a front panel 54. Then the operator moves the card along a card striper 56 to relate the saccharimeter reading to the corresponding sample in the control unit. The card is then transferred to a bin 58, and the pot of remaining material along a conveyor 60 to a waste bin 62.

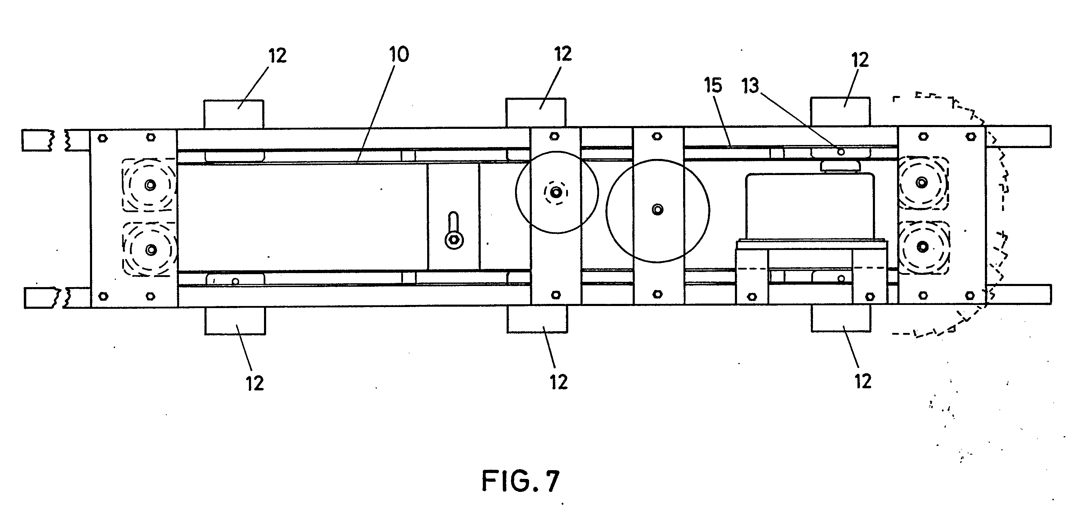

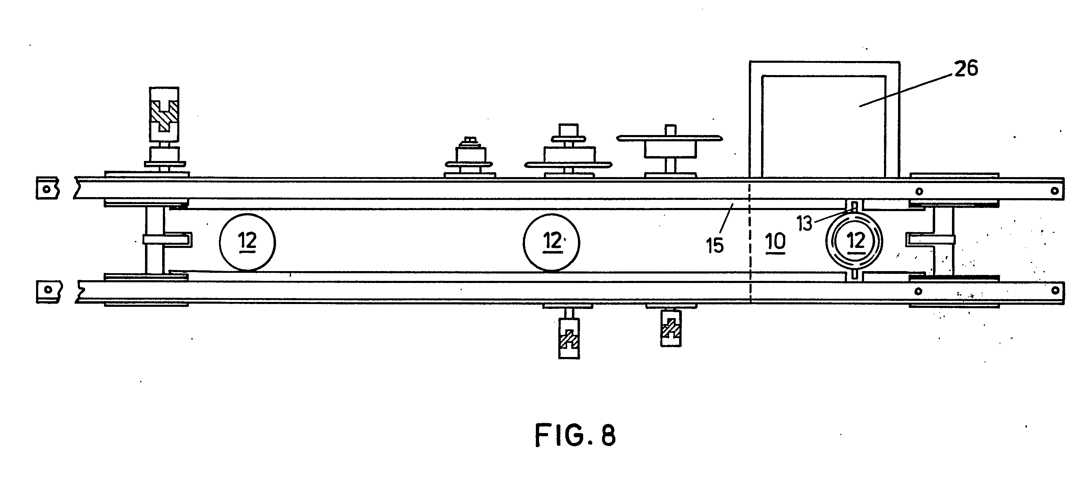

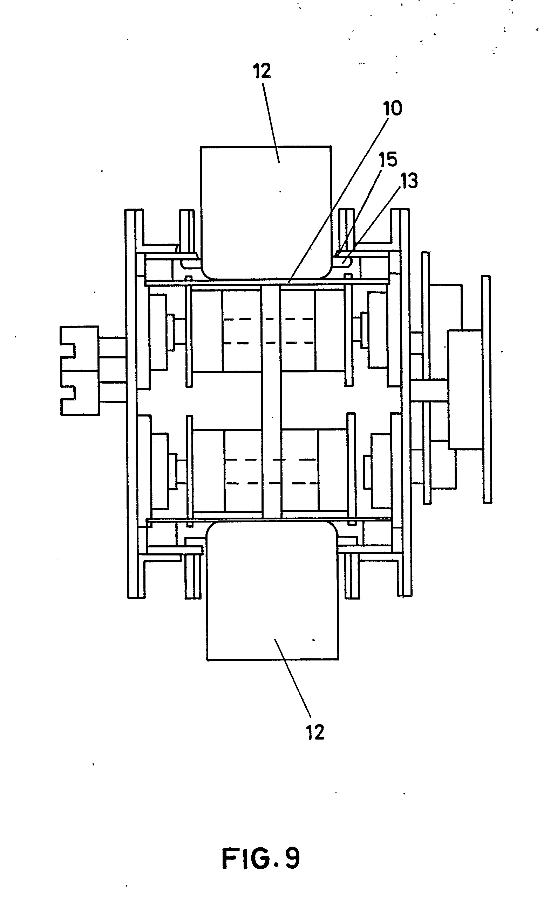

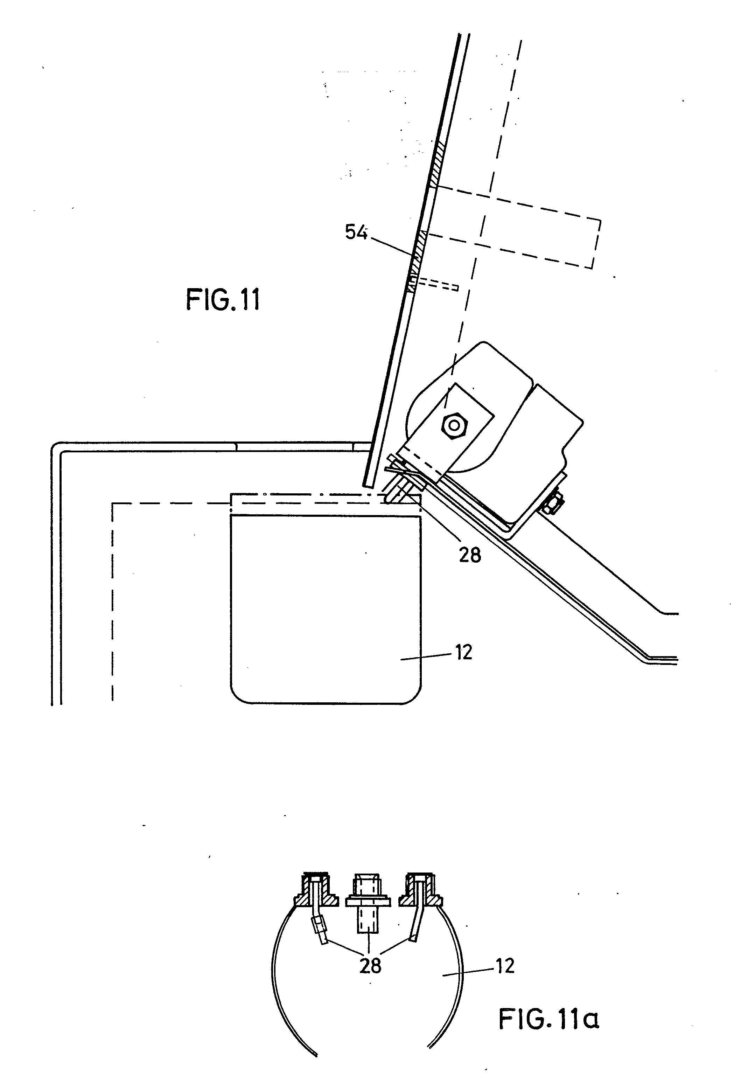

With reference to Figures 7, 8 and 9 it can be seen that the containers 12 have lugs 13 projecting to each side. The lugs 13 cooperate with brackets 15 to form clips which hold the containers 12 on the conveyor 10. At the fill station 14, there are gaps in the brackets 15, so the container 12 can be lifted off the conveyor 10 from a position shown in solid lines to a position shown in broken lines in Figure 11. This is done by a balance head 120 (Figure 10) which moves upward, lifts the container 12 out of its retaining clips, and tares the container 12 to zero.

The balance head 120 has screwed thereto a pan platform 122 which carries a compensating weight 124, and above that an anti-spill skirt 126 and a non-slip pad 128. The weight 124 projects through a hole in a balance cover 130 so that the pad 128 can contact the base of a container 12 through a link in the conveyor 10 at the fill station 14. The balance 26 also comprises a main base 132, a sub-base 134, and a lift platform 136 supported on four return springs 138 and guided by two pins 140. A motor 142 is secured to the base 132, and serves to raise and lower the platform 136 between down and up stop microswitches 144, 146. The motor 142 actually lifts the platform 136 by rotating a cam 148 which contacts an underside of the platform 136. The rotation is effected on commands from the control unit through gear wheels 150 and a shaft 152 fast on the cam 148. The balance head 120 is fastened to the lifting platform 136 by three screws 154 from below.

On the arrival of a container 12 at the fill station 14, a photo-electric cell (not shown) notifies the control unit, and the motor 142 raises the lift platform 136 to the up stop position determined by the microswitch 146. The pad 128 consequently lifts the container 12, which is automatically tared by the balance 26. The operator places a sample (20 to 28 g) of matter (brei) in the container 12. The balance calculates how much reagent (lead acetate) is required by an internal program. Control valves governing nozzles 28 (slow, fast and medium from the left to right in Figure 11a) are opened in sequence so that the exact amount of reagent required is added according to the balance. The motor 142 then lowers the container 12 back into its clips, and the platform 136 to the down stop position (microswitch 144). The conveyor 10 advances a step so that the container 12 is moved to the second or mix station 16 while an empty container is brought to the fill station 14.

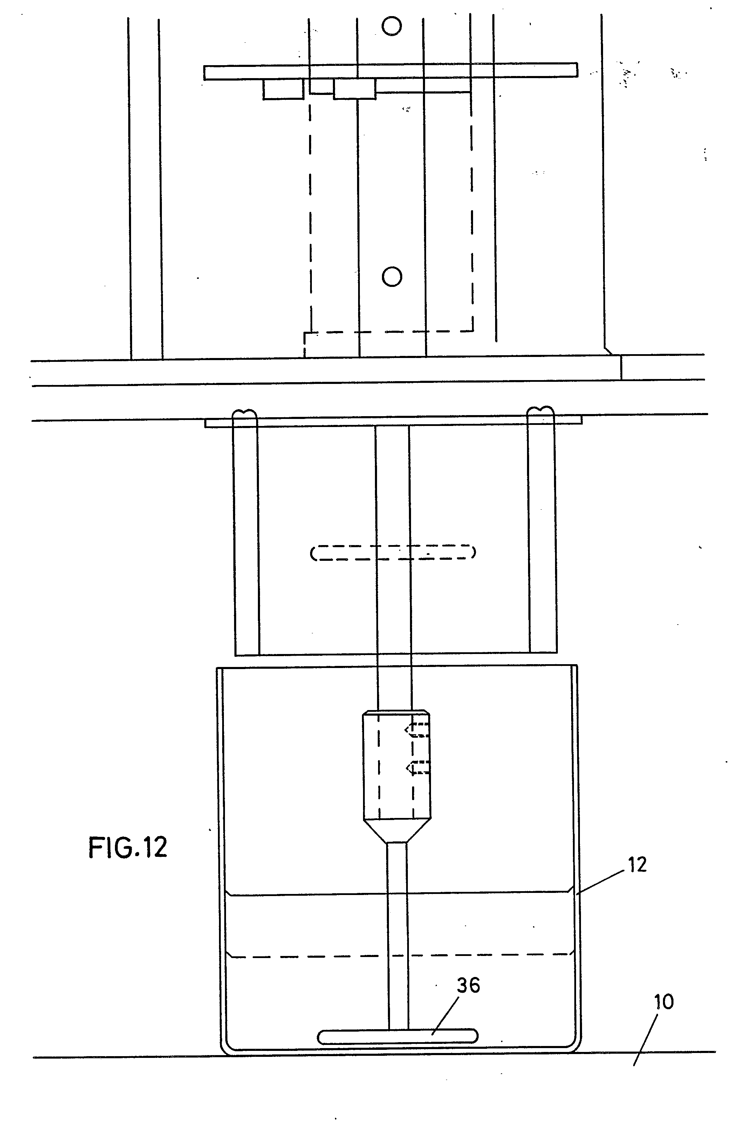

At the mix station 16 (Figure 12), a direct drive T-bar stirrer 36 is lowered from an upper or rest position shown in broken line into the container 12 by means of a motor (not shown) and the control unit along a guide 38. The stirrer 36 is rotated for a period, and then raised again to its rest position. When the conveyor 10 advances a further step, the container 12 is moved to the third or sample station 18.

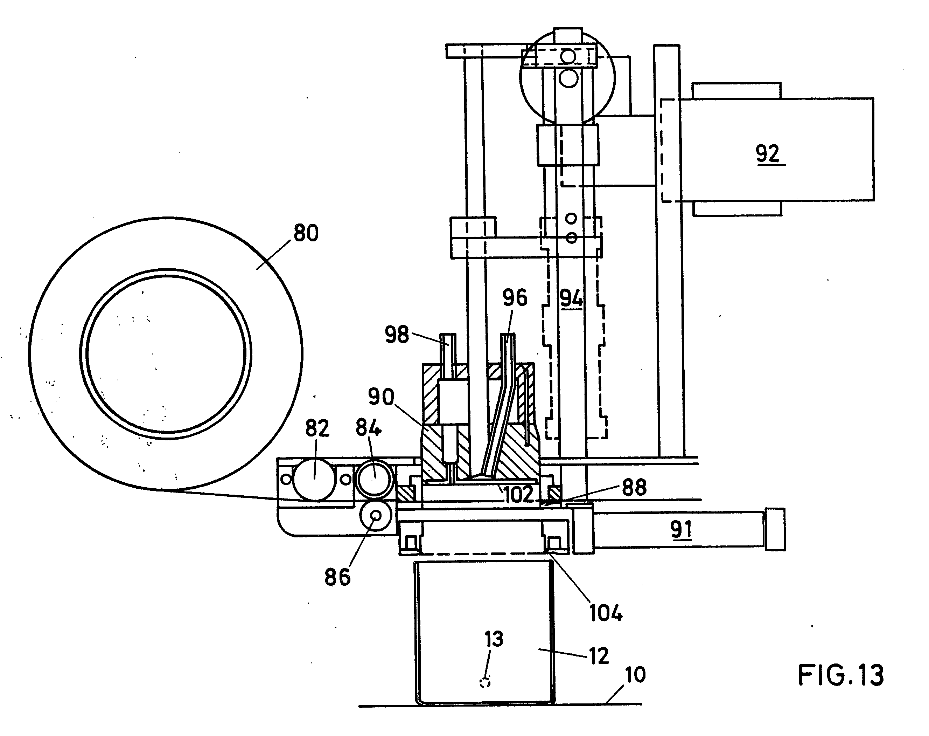

Figure 13 shows means for drawing off a sample from a container 12 at the third or sample station 18 of the apparatus. A roll 80 of filter paper is rotatably mounted to the left. The filter paper passes under a rotatable roller 82 and a clamp roller 84 for controlling the movement of the filter paper towards the right in synchronism with other parts of the apparatus. Below the clamp roller 84 is a drive roller 86 for advancing the paper by a motor on a command from the control unit. The filter paper passes over a die plate 88 where a disc is cut out of the paper by a downward movement of a punch 90. The disc of filter paper is retained on the surface of the punch by vacuum and is carried by further movement of the punch 90 down onto the surface of liquid in the container 12. The movement of the punch 90 is controlled by a variable speed motor 92 down and up a guide post 94. A sample is sucked up through a tube 96 and pumped away by means (not shown) for examination in a saccharimeter and/or colourimeter. The punch 90 is then raised by the motor 92, and the used filter paper blown off by a jet of air through a tube 98 from a gauze 102. As the gauze 102 passes a row of air blow off nozzles 104 above the container 12, any liquid remaining on the head of the punch 90 is removed. Waste filter paper not cut out by the punch 90 is removed by a waste paper conveyor 91. The container 12 is then conveyed towards the wash station 20.

## Claims

1. Liquid sampling apparatus characterized by a conveyor (10) for transporting containers (12) for liquid through a number of stations (14, 16, 18) in sequence, a balance (26) at a first station (14) for determining the weight of matter in the container (12), means (28) for introducing into the container (12) an amount of reagent corresponding to the weight of matter, a mixer (36) at a second station (16) for stirring the contents of the container (12), means (96) for drawing off a sample of the contents of the container (12) at a third station (18), means for examining the sample, and means for washing and drying the container at subsequent stations (20, 22, 24).

2. Apparatus according to claim 1 in which the conveyor (10) is a twin chain conveyor provided with mountings (15) for securing the containers (12).

3. Apparatus according to claim 1 or claim 2 in which the first, second and third stations (14, 16, 18) are in line, and the washing (20) and drying (22, 24) stations on a return run of the conveyor (10 below the earlier stations (14, 16, 18).

4. Apparatus according to any preceding claim in which the conveyor (10) is advanced intermittently by a control unit taking into account the state of the operations performed at the various stations.

5. Apparatus according to any preceding claim in which the presence of sample is noted by photoelectric cells on an identifying card conveyor.

FIG.1

FIG.2

FIG. 3

FIG.4

FIG. 5

FIG.6

EP 0 364 078 A1

FIG. 7

EP 0 364 078 A1

FIG. 8

EP 0 364 078 A1

FIG. 9

FIG.10

FIG.11

54

28

12

FIG.11a

28

12

FIG.12

12

36

10

FIG.13

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | DE-A-1 698 229 (INGENJÖRSFIRMAN NILS WEIBULL, AB)<br>* Pages 1,2; page 3, line 9 - page 4, line 6; page 6, lines 10-14; page 8, lines 8-23; page 9, lines 5-12; page 12, line 22 - page 13, line 12; page 17, lines 17-23 * | 1,3,4 | G 01 N 35/02<br>G 01 N 35/04 |
| X,D | DE-A-1 907 464 (DEUTSCHE SEMPERIT GUMMIWERK GmbH)<br>* Figures 3,6-8; page 3, lines 16-31; page 4, line 23 - page 6, line 6 * | 1 | |
| A,D | US-A-3 481 709 (T.J. SLONE)<br>* Abstract; figure 1 * | 1,3 | |
| A,D | US-A-3 843 323 (B.A. QUAME)<br>* Figures 1,3,5 * | 1-3 | |
| A,D | FR-A-2 537 552 (A.G.E.I., S.A.)<br>* Abstract; page 3, lines 8-11,33-35 * | 5 | TECHNICAL FIELDS SEARCHED (Int. Cl.5)<br><br>G 01 N 35/00 |

The present search report has been drawn up for all claims

| Place of search | Date of completion of the search | Examiner |
|---|---|---|
| THE HAGUE | 20-09-1989 | HODSON C.M.T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)